(19) Europäisches Patentamt
European Patent Office
Office européen des brevets

(11) Publication number : **0 521 674 A1**

## (12) EUROPEAN PATENT APPLICATION

(21) Application number : **92305952.1**

(22) Date of filing : **29.06.92**

(51) Int. Cl.$^5$ : **C12N 5/06,** A01K 61/02, C12N 5/00

(30) Priority : **01.07.91 US 723870**
**24.10.91 US 782840**

(43) Date of publication of application :
**07.01.93 Bulletin 93/01**

(84) Designated Contracting States :
**AT BE CH DE DK ES FR GB GR IT LI LU MC NL PT SE**

(71) Applicant : **Dominko, Tanja**
**2320 White Oak Trail**
**Oregon, Wisconsin 53575 (US)**

(72) Inventor : **Dominko, Tanja**
**2320 White Oak Trail**
**Oregon, Wisconsin 53575 (US)**

(74) Representative : **West, Alan Harry et al**
**R.G.C. Jenkins & Co. 26 Caxton Street**
**London SW1H 0RJ (GB)**

(54) **In vitro maturation of denuded bovine oocytes.**

(57) The present invention is directed to a process for maturing denuded bovine oocytes. The process includes stripping cumulus cells from the oocyte and allowing the denuded oocyte to mature in a conditioned medium including maintenance medium having therein a monolayer of cumulus cells for a period of approximately 10-24 hours, and preferably 16-20 hours. The process can also be adapted to an oocyte screening process for selecting bovine oocytes which make better candidates for fertilization and embryo development. The process includes observing the development and ejection of the polar body. This phenomenon has direct correlation with the developmental potential of the bovine oocyte.

EP 0 521 674 A1

The present invention is generally directed to in vitro oocyte maturation, and is specifically directed the in vitro maturation of denuded bovine oocytes. Additionally, the present invention is directed to a method for pre-selecting high quality bovine oocytes, i.e., oocytes which have a higher probability of developing into viable embryos upon fertilization, for use in nuclear transfer or other genetic manipulation techniques.

The present invention is also directed to a process for maturing cumulus enclosed oocytes having higher developmental competence.

Technologies to increase the supply of bovine embryos by cloning and their genetic alteration by gene transfer are near commercial use. These technologies require: (1) mature (metaphase II) oocytes in vitro, which upon enucleation serve as recipient cells for nuclear transfer; (2) the production of mature (metaphase II) oocytes, which upon fertilization develop into viable embryos and can be used as nuclear donors in nuclear transfer; and (3) the ability to culture the embryos in vitro from fertilization to a stage compatible with the transfer to the uterus of a recipient cow, commonly in the morula or blastocyst stage.

For the successful commercial use of techniques such as genetic engineering or cloning, it must be possible to obtain and mature oocytes in vitro before they can be used as recipient cells for nuclear transfer or before they can be fertilized by the sperm cell to develop into an embryo. This process generally requires collecting immature (prophase I) oocytes from bovine ovaries obtained at a slaughterhouse and maturing the oocytes in a maturation medium prior to fertilization or enucleation.

Oocytes are normally matured and protected in a mass of cumulus cells, i.e., non-reproductive or somatic cells which surround the oocyte and are believed to provide both protection and nutrients needed to mature the oocyte. The presence of cumulus cells, however, creates a cloud around the oocytes making it very difficult if not impossible to observe oocytes during the maturation period. As such, it is difficult to determine nuclear maturity of the cumulus-enclosed oocyte.

In one study of oocyte maturation in the mouse, the cumulus cells were stripped away from a mouse oocyte (Schroeder and Eppig, 1989 Developmental Biology, Vol. 102, pp. 493-497). While the researchers determined that a denuded mouse oocyte could be matured in a specific maturation medium, there was no apparent utility or economic value associated with this study.

Similar studies have been conducted with bovine oocytes. However, maturation protocols for denuded bovine oocytes yielded a lower number of embryos developing from these oocytes. Barnes, in PCT patent application No. 9013627, studied the in vitro maturation of bovine oocytes. In this process, Barnes collected cumulus enclosed oocytes (CEOs) from bovine ovaries, matured them in a maturation medium for an extended period of time, stripped the cumulus cells from the oocytes (denuding), and attempted to maintain the oocytes in a preparation containing a co-culture of oviductal cells and media. The oocytes were denuded after the maturation process.

Although these studies were performed with oocytes stripped of the cumulus cell layer, i.e., denuded oocytes, no method has yet been ascertained for selectively screening oocytes in order to obtain oocytes which would be primary candidates for embryo development.

It is therefore an object of the present invention to develop a method for the maturation of denuded oocytes.

It is another object of the present invention to develop a method for studying denuded oocyte maturation.

It is further an object to develop a screening method to determine, by in vitro means, which bovine oocytes have the highest probability of becoming successfully fertilized and developing into viable embryos. Presently, it is estimated that only about 20 to 30% of bovine oocytes, which are matured in vitro in a maturation medium, will successfully develop into embryos.

It is also an object of the present invention to increase the frequency of developmentally competent bovine oocytes.

Further, it is an object of the present invention to develop a conditioned medium which enhances the rate of oocyte development.

Another object of the present invention is to investigate the relationship between the kinetics of polar body formation (i.e., the completion of the meiosis I phase) and the presence of gonadotropins and their effect on fertilization and development of cumulus enclosed oocytes.

These objects and others are addressed by the present invention which is directed to a process of maturing denuded bovine oocytes comprising obtaining and isolating bovine cumulus enclosed oocytes (CEOs) from bovine ovaries, placing the CEOs in a maintenance medium, removing (stripping) the cumulus cells from the oocytes ("denuding" oocytes), and maturing the denuded oocytes in a conditioned medium containing a previously formed monolayer of cumulus cells for about 10-24 hours.

The present invention is also directed to a process for selecting bovine oocytes of higher quality for fertilization, nuclear transfer and other genetic manipulation processes comprising the above-referenced steps and observing the presence and ejection (or shedding) of the first polar body. Genetic manipulation is a procedure of inserting and/or deleting genes, parts of genes, fragments of DNA and/or RNA, or inserting or deleting the entire genome. Genetic manipulation includes nuclear transfer processes, gene transfer, embryo cloning and genetic alteration by gene transfer. It has been determined that there is a direct correlation with those oocytes that eject their polar bodies early and embryo development if a specific maturation protocol is used.

The present invention is also directed to a specific conditioned medium for maturing denuded bovine oocytes comprising a maintenance medium in which a monolayer of cumulus cells has been allowed to develop and to condition the medium.

Advantageously, the present invention provides a method for screening bovine oocytes before fertilization or enucleation by observing the ejection of polar bodies. Thus, the observation of the first polar body on the oocyte can be used to predict a suitable candidate for fertilization or enucleation. The present invention increases the frequency of obtaining developmentally competent oocytes.

Without wishing to be constrained to one explanation, it is believed that the ejection of the first polar body from the oocyte coincides with nuclear maturation of the oocyte. Those oocytes which eject polar bodies early are believed to be more prone to fertilization and/or activation after nuclear transfer and subsequent to embryo development.

At about 10-16 hours development under the conditions of the present invention, the oocytes have been found to be healthier and more viable and more prone to cleavage, fertilization and development to the blastocyst stage. Thus, the primary purpose of the present invention, i.e., obtaining healthy oocytes, is achieved by maturing the denuded bovine oocytes in a conditioned medium containing a monolayer of cumulus cells for approximately 10-24 hours. Preferably, the maturation period lasts approximately 16 hours, after which the oocytes are removed from the conditioned medium, maintained in maintenance medium for about 8 hours and fertilized.

The present invention is also directed to the addition of luteinizing hormone, or a hormone structurally and chemically similar to luteinizing hormone, in a maintenance medium containing CEOs to improve the selection of developmentally competent oocytes by decreasing the time required for polar body formation of the CEOs. The polar body formation on the oocytes is noticed at around 10-16 hours maturation in the medium.

This technique involves selecting bovine oocytes without initially denuding the oocyte. In this process the CEO is isolated and matured in a maintenance medium containing luteinizing hormone for approximately 10-24 hours, preferably about 14-22 hours, most preferably about 16-20 hours, and specifically about 16 hours. After maturation, the cumulus cells are removed and the oocytes are observed for the presence of a first polar body. The bovine oocytes are selected based upon this observation.

Further objects, features and advantages of the invention will be apparent from the following detailed description of the invention.

Fig. 1 is a bar graph illustrating the percent polar body appearance at 16, 20 and 24 hours during maturation for cumulus-enclosed oocytes (CEO) and for non cumulus-enclosed oocytes (NO) in Example 2.

Fig. 2 is a bar graph illustrating the percent normal fertilization by time of polar body formation at 16, 20 and 24 hours for cumulus-enclosed oocytes (CEO) and for non cumulus-enclosed oocytes (NO) in Example 2.

Fig. 3 is a bar graph illustrating the percent cleavage of oocytes classified by the time of polar body formation at 16, 20 and 24 hours for cumulus-enclosed oocytes (CEO) and for non cumulus-enclosed oocytes (NO) in Example 2.

Fig. 4 is a bar graph illustrating the day 8 blastocyst formation of oocytes classified by time of polar body ejection at 16, 20 and 24 hours for cumulusenclosed oocytes (CEO) and for non cumulus-enclosed oocytes (NO) in Example 2.

For purposes of the present invention, the term "oocyte" is used to define an egg cell which undergoes meiosis to form an ovum (e.g., an unfertilized, nonmotile female gamete) and a first polar body. Polar bodies are smaller cells that are produced during meiosis in oogenesis (the formation of an ovum) and ejected from maturing oocytes after completion of the first meiotic (reduction division) stage, also termed meiosis I. Oocyte maturation is defined as the progression of the primary oocyte through meiosis I until the development stage is arrested at the metaphase stage of meiosis II. The oocyte, now a secondary oocyte, remains in this arrested stage until fertilization triggers the completion of meiosis II (Alberts, B., et al., Ed., 1989, Molecular Biology of the Cell, Second Edition, Garland Publishing, Inc., New York, pp. 859-863). Therefore, the first meiotic division occurs in a primary oocyte and generates the secondary oocyte. This then undergoes the secondary meiotic division to produce the ovum.

The process of the present invention is specifically directed to the in vitro maturation of denuded oocytes prior to fertilization and to the selection of those oocytes considered to be primary candidates for fertilization or enucleation. For purposes of the present invention, these oocytes are also termed "developmentally competent oocytes." Used here, the term "developmentally competent oocytes" refers to oocytes having the ability to develop embryos.

With the advent of the present invention, a benchmark has been provided for determining the maturity of an oocyte, which is necessary for normal fertilization and embryo development. This benchmark is the observation of the formation and ejection of the polar body in the oocyte.

The importance of the observation of the polar body is explained as follows. The female gamete should have a haploid number of chromosomes before it can become fertilized. Prior to fertilization, the female gamete must undergo reduction division to establish the proper number of chromosomes. The polar body develops and is ejected after completion of the first meiotic division, i.e., meiosis I. At this point, the polar body contains half of the chromosomes originally present in the oocyte. The oocyte contains the other half. When the first polar body has been ejected, the oocyte has acquired the required haploid number of chromosomes. Upon fertilization by the sperm cell, the complement haploid sperm chromosomes are added enabling the formation of the diploid zygote-embryo.

In the past, it was not possible to determine the stage of oocyte development by observing the oocyte, primarily because in vitro maturation required the presence of cumulus cells, which surrounded the oocyte and hid it from observation. Therefore, following the kinetics of the first polar body formation and its relation to the oocyte's developmental competence was not possible.

By the development of the present invention, the requirement for a surrounding mass of cumulus cells is no longer needed. The oocyte can be seen and the presence of the first polar body can be determined.

## Maintenance Medium

There are a variety of embryo culture and maintenance media routinely used for the collection and maintenance of embryos, and specifically bovine embryos. Examples of known media, which may be Used for bovine embryo culture and maintenance, include Ham's F-10 + 10% fetal calf serum, Tissue Culture Medium-199 (TCM-199) + 10% fetal calf serum, Tyrodes's-Albumin-Lactate-Pyruvate (TALP), Dulbecco's Phosphate Buffered Saline (PBS), Eagle's and Whitten's media. One of the most common media used for the collection and freezing of embryos is TCM-199 and 1 to 20% serum supplement including fetal calf serum, new born serum or steer serum. A preferred maintenance medium includes TCM-199 with Earl salts, 10% fetal calf serum, 0.2MM Na-pyruvate and 50 ug/ml gentamicin sulphate.

Another maintenance medium is described in U.S. Patent 5,096,822 to Rosenkrans, Jr. et al., entitled "Bovine Embryo Medium," which is incorporated herein by reference. This embryo medium, named CR1, contains the nutritional substances necessary to support an embryo.

CR1 contains hemicalcium L-lactate in amounts ranging from 1.0 mM to 10 mM, preferably 1.0 mM to 5.0 mM. Hemicalcium L-lactate is L-lactate with a hemicalcium salt incorporated thereon. Hemicalcium L-lactate is significant in that a single component satisfies two major requirements in the culture medium: 1) the calcium requirement necessary for compaction and cytoskeleton arrangement; and 2) the lactate requirement necessary for metabolism and electron transport. Hemicalcium L-lactate also serves as valuable mineral and energy source for the medium necessary for viability of the embryos.

Advantageously, CR1 medium does not contain serum, such as fetal calf serum, and does not require the use of a co-culture of animal cells or other biological media, i.e, media comprising animal cells such as oviductal cells. Biological media can sometimes be disadvantageous in that they may contain micro-organisms or trace factors which may be harmful to the embryos and which are difficult to detect, characterize and eliminate.

Examples of the main components in CR1 medium include hemicalcium L-lactate, sodium chloride, potassium chloride, sodium bicarbonate and a minor amount of fatty-acid free bovine serum albumin. Additionally, a defined quantity of essential and non-essential amino acids may be added to the medium. CR1 with amino acids is known by the abbreviation "CR1aa."

CR1 medium preferably contains the following components in the following quantities:

| | |
|---|---|
| sodium chloride | - 114.7 mM |
| potassium chloride | - 3.1 mM |
| sodium bicarbonate | - 26.2 mM |
| hemicalcium L-lactate | - 5 mM |
| fatty-acid free BSA | - 3 mg/ml |

## Conditioned Medium

In order for denuded bovine oocytes to mature, the maintenance medium must be conditioned. The term "conditioned," as used herein, refers to the action of the cumulus cells on the maintenance medium to provide a desirable environment for the denuded bovine oocytes. By allowing the cumulus cells to dwell in the maintenance medium for a period of time to condition the medium, approximately two days, the medium and the cells will support maturation of denuded bovine oocytes. Because the bovine oocytes are denuded, i.e., stripped of cumulus cells, the quality of the oocytes can then be determined by the procedures described herein.

A preferred conditioning process is as follows. Cumulus cells are stripped from an oocyte mechanically by pipetting CEOs through the neck of the micropipette (180-210 $\mu$m inner diameter) attached to a syringe. Cumulus cells fall off and denuded oocytes are removed from the maintenance medium. Cumulus cells are further disaggregated mechanically by pipetting them through the neck of the micropipette (20-30 $\mu$m inner diameter).

Other methods of stripping cumulus cells from an oocyte include removing the cells by vortexing approximately 1 ml of the CEO medium for approximately 2-2½ minutes. Alternatively, the cells may be mechanically stripped by ultrasound techniques known to the art. The cells may also be stripped enzymatically by the application of proper enzymes such as trypsin or collagenase according to methods known to the art of cell culture.

The medium with disaggregated cells is then placed into 5 ml of maintenance medium in a conical tube. Cells are washed twice according to the following procedure: The tube is centrifuged at 3000 rpm for 15 minutes. The supernatant is discarded and 5 ml of fresh medium is added to the pellet of cells at the bottom of the tube.

The pellet obtained after the second washing is resuspended in the maintenance medium to a final concentration of $1 \times 10^7$ cells/ml. 50 $\mu$l drops are made from the cell suspension, and covered with paraffin oil and placed in an incubator for 2-4 days (incubation conditions: 5% $CO_2$ in air, 39°C, humidified atmosphere) to form a primary cell culture layer. A primary cell culture layer is defined as a layer mostly comprising cumulus cells from the original tissue. These cells have the same functioning characteristics as cumulus cells in vivo. After two days, the drops are used for the maturation of denuded bovine oocytes.

In one embodiment, the present invention can operate as follows:

CEOs are collected from the small antral follicles from slaughterhouse bovine ovaries. The CEOs are placed in the maintenance medium and stripped from surrounding cumulus cells. A description of the stripping procedure is detailed elsewhere in this application.

Denuded oocytes are then washed twice in Hepes-buffered Tyrode medium and place in conditioned medium drops, prepared two days earlier in which a cumulus cell monolayer is formed. The oocytes are matured for approximately 10-24 hours, preferably about 12-22 hours, more preferably about 16-20 hours and most preferably about 16 hours, at 39°C, with 5% $CO_2$ in air and maximal humidity.

Following this maturation time, the oocytes are examined microscopically (40x) for the presence of the first polar body. The oocytes which have released the first polar bodies are then considered prime candidates for fertilization. The oocytes may be fertilized in a modified tyrodes medium, known to the art, for 48 hours and placed in a culture medium for further development.

## Maturation of CEOs

It has also been found that the rate of CEO development, as opposed to denuded oocyte development, is greatly enhanced by the addition of a luteinizing hormone to the maintenance medium. Luteinizing hormone is a glycoprotein secreted by the pars distalis of the pituitary. In females, the hormone promotes the maturation of the Graafian follicles with the production of estrogens and is essential for ovulation and the formation of the corpora lutea. For purposes of this invention, the term "luteinizing hormone" or "LH" refers to any compound with LH activity. Such compounds include naturally-derived LH, human chorionic gonadotropin (HCG) from the human placenta, and synthetic compounds expressing LH activity.

The medium requires only enough luteinizing hormone to enhance the rate of oocyte development. Thus, luteinizing hormone can be included in the conditioned medium in an amount from 2 $\mu$g/ml to 10 $\mu$g/ml, and preferably about 5 $\mu$g/ml.

Reference is now made to the following examples. While the examples are not intended to be limiting in any way to the present invention, they provide a description of the best mode of the invention.

## EXAMPLE 1

The object of this example was to test whether somatic cell (cumulus) monolayers with conditioned medium could support the maturation of denuded oocytes.

CEOs were collected from small antral follicles (2-6mm) from slaughterhouse ovaries. The oocytes were matured according to one of the following treatments:

1. CEOs were placed in maturation medium (TCM-199, supplemented with 10% FCS, pyruvate, gentamicin, 5 $\mu$g/ml LH and 1 $\mu$g/ml estradiol). This medium is also called TCM-199 -- Hormones.

2. Oocytes were manually stripped free of cumulus cells (NO) and placed in TCM-199 -- Hormones.

3. NO were placed in conditioned medium (maintenance medium conditioned for two days by cumulus cells).

The conditioning medium was prepared by washing the cumulus cells, suspending them in 50 microliter drops of maintenance medium and allowing them to form monolayers to condition the medium for two days.

Oocytes in all treatments were matured in 50 microliter drops under paraffin oil (10 oocytes/drop) for 21 hours at 39°C, 5% $CO_2$ in air at maximal humidity. After maturation all oocytes were examined for the presence of first polar bodies.

Oocytes were fertilized in modified tyrodes medium for 48 hours and placed in CR1aa for further development.

The results of this experiment are illustrated in the following Table 1.

## TABLE 1

| Maturation Treatment PI | No. | Polar Body 21h | No. (%) | CLV(%) 48h PI | BL% 8 days |
|---|---|---|---|---|---|
| CEO in TC199- hormones | 172 | + − | 114(66)[a] 58(34)[b] | 93(82)[a] 19(32)[b] | 29(31)[b] 7(37)[b] |
| NO in TC199- hormones | 199 | + − | 125(63)[a] 74(37)[b] | 33(26)[b] 22(20)[b] | 3( 9)[c] 0( 0)[c] |
| NO on mono- layer in TC199 | 150 | + − | 88(59)[a] 62(41)[b] | 73(83)[a] 31(50)[b] | 42(58)[a] 8(26)[b] |

PI = postinsemination; BL = blastocysts; CLV = cleavage

[a,b,c] Means with different superscripts differ (P<0.05)

The term "cleavage" (CLV) defines the number of oocytes divided at least once after being fertilized. Eighty-two percent of the CEOs in the first treatment had cleavage, while only 26% in the second treatment (NO monolayer) cleaved. However, 83% in the third treatment (with a cumulus monolayer) cleaved.

Although there is substantially no difference between the first and third treatments in terms of cleavage, reference is now made to the blastocyst development column for distinction. Of those cells which cleaved in the first treatment, on day 8 only 31% formed blastocysts. However, 58% in the third treatment formed blastocysts. These results suggest that the cumulus cell monolayer with two days conditioned medium can support maturation of denuded bovine oocyte. Further, using this system, the presence of a polar body can be used as an indicator of the oocyte's developmental potential.

## EXAMPLE 2

The object of this example was to determine the oocytes with highest embryo development competence.

Oocytes were obtained and matured according to the procedure of Example 1 for maturation of denuded oocytes (NO) and cumulus enclosed oocytes (CEO). CEOs were matured in TCM-199-hormones for 16 hours. At this time, they were manually stripped free of cumulus cells. They were examined for the presence of first polar bodies (PB) at 16, 20 and 24 hours after the start of conditioning. NO were stripped free of cumulus cells prior to culture and matured on cumulus monolayers (2 days old) in TC199-hormones. NO were examined for the presence of first polar bodies at 16, 20 and 24 hours after the start of culture.

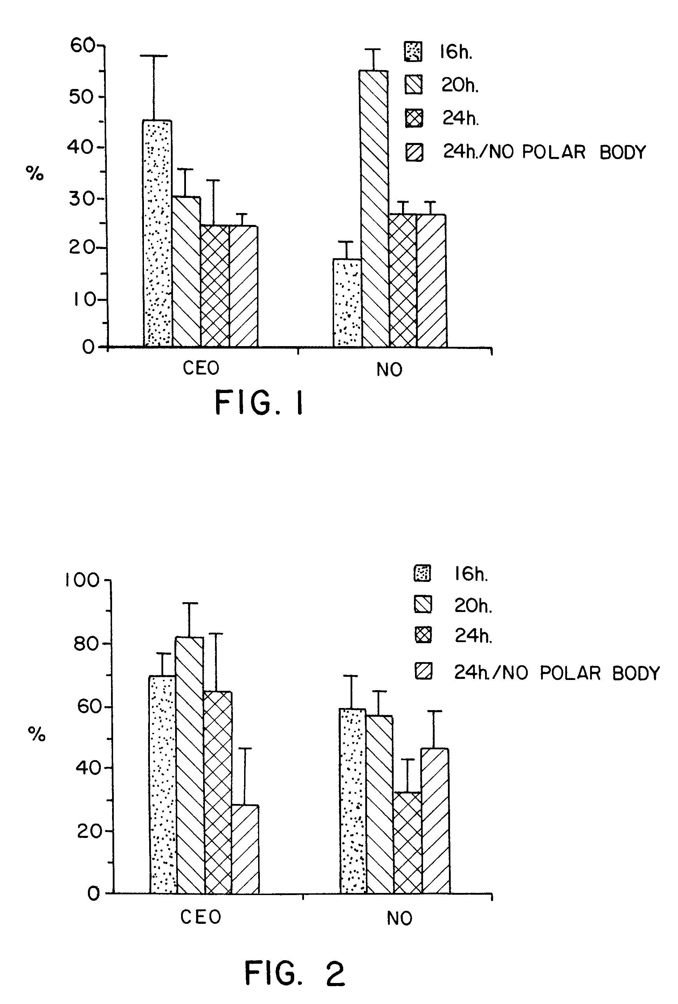

At 24 hours, all oocytes were fertilized (each replicate with semen from a different bull) in Tyrode's medium for 48 hours. At this time, all oocytes that cleaved at least once were transferred to CR1aa medium for development for 6 days to day 8 blastocyst stage. The results are summarized in Figs. 1-4 and below in Table 2.

## Table 2

## Development of Oocytes Classified by Time of PB Ejection

| Treatment | | %PB±SE | %NF±SE | %CLV±SE | %d8BL±SE |
|---|---|---|---|---|---|
| CEO | 16hr | 45.3±13.1 | 69.7±6.9 | 46.4±20.5 | 43.4±23.0 |
| | 20hr | 30.3±5.6 | 82.1±10.6 | 66.4±4.6 | 31.4±5.2 |
| | 24hr | 24.4±8.9 | 65.2±17.9 | 60.9±10.8 | 15.8±8.9 |
| NO | 16hr | 18.1±3.3 | 59.5±10.5 | 86.7±2.1 | 54.7±5.8 |
| | 20hr | 55.1±4.3 | 56.9±8.0 | 76.7±2.5 | 37.3±3.8 |
| | 24hr | 26.8±2.5 | 32.3±10.4 | 65.7±11.0 | 24.8±5.2 |

PB – polar body; NF – normal fertilization;
CLV – cleavage at 48 hours; d8BL – day 8 blastocyst.

The results suggest that there are differences in timing of PB ejection that depend on whether oocytes were matured in the presence (CEO) or absence (NO) of cumulus cells. The majority of oocytes in the CEO group had a PB ejected at 16 hours while the NO group exhibited the highest proportion of PB at 20 hours. Most of these oocytes had polar bodies by 20 hour post-maturation (75% CEO and 72% NO). As illustrated in Fig. 2, normal fertilization was slightly higher in CEO oocytes.

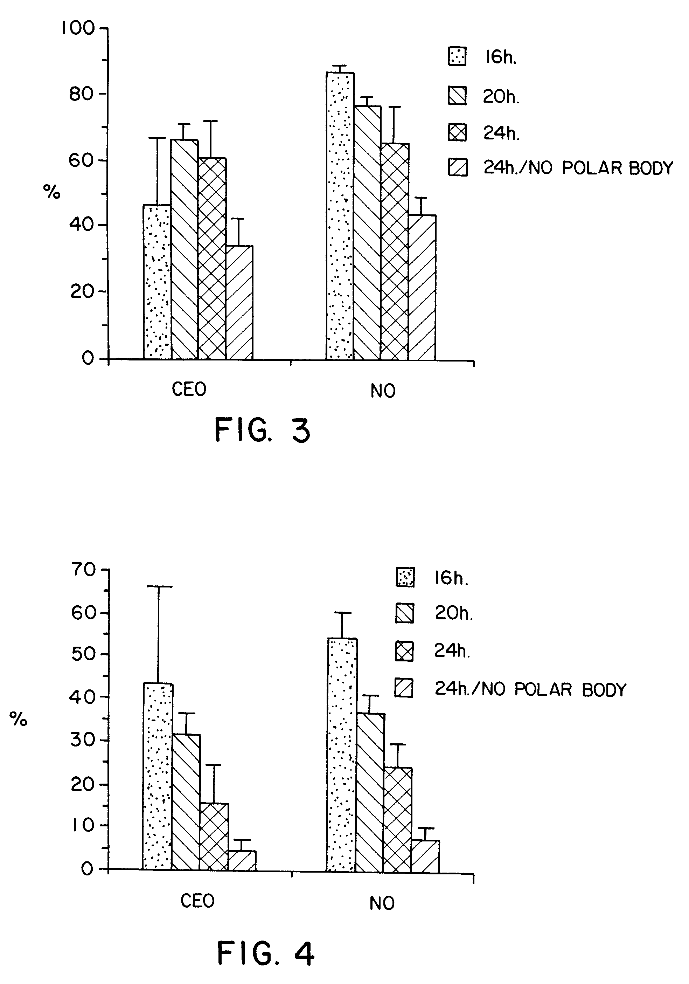

The cleavage rates were higher in the NO group no matter when the polar body was ejected. Cleavage was highest in 16 hour oocytes (85%) and then declined slowly as oocytes had the PB ejected later. This is illustrated in Fig. 3. As illustrated in Fig. 4, the same tendency was evident from results of day 8 blastocyst development. Thus, it is believed that oocytes that had PBs cleaved earlier and developed at higher rates in both groups (CEO and NO).

EXAMPLE 3

The large variation (SE) in CEO at 16 hours, as illustrated in Fig. 1, was believed to be due to different gonadotropins used during maturation in different replicates. This example was developed to determine the effect of different gonadotropins on PB ejection from CEOs.

The process of obtaining and maturing the oocytes in Example 2 was followed with the following distinctions. Only evenly granulated oocytes with compact cumulus investment were used for maturation. Intact cumulus complexes were matured in TCM-199, supplemented with 5 µg/ml ovine-derived LH (National Institute of Arthritis, Diabetes and Digestive and kidney Diseases-National Institute of Health) (NIH-oLH) (biological potency: 2.3 Units/mg) and 1 µg/ml estradiol (Treatment oLH) or 25 µg/ml porcine-derived FSH (Schering) (FSH-p) and 1 µg/ml estradiol (Treatment FSH-p). After 16 hours of maturation, oocytes were manually stripped free of cumulus cells and placed in TCM-199. They were examined for the presence of the first polar body at 16, 20 and 24 hours. Oocytes not expelling polar bodies by 24 hours of conditioning were not included in the study (23-25% of all oocytes). All oocytes were fertilized in glucose-free TALP at 24 hours. Oocytes that cleaved at least once (48 hours after fertilization) were placed in CR1aa for further development. Data were analyzed using a completely randomized design with 2 x 3 factorial combinations. Each treatment was replicated (n = 4).

The results are summarized in Table 3 as follows:

## Table 3

| Maturation Treatment | Age of Oocytes | No. of Oocytes | % Polar Body±SE | % Cleaved±SE ($\geq 2$ cells) | % Blastocysts/ /Cleaved +SE (day 8) |
|---|---|---|---|---|---|
| oLH | 16 | | 66.0±2 | 77.8±3[a] | 47.3±2[a] |
| | 20 | 568 | 17.1±4 | 64.3±9[a,b] | 35.4±3[b] |
| | 24 | | 15.6±3 | 48.4±6[b] | 9.6±3[c] |
| FSH-p | 16 | | 26.5±6 | 43.5±10[b] | 33.7±16[b] |
| | 20 | 479 | 43.8±3 | 55.2±6[b] | 29.6±3[b] |
| | 24 | | 28.4±7 | 39.2±13[b] | 11.8±13[c] |

[a,b,c]Means with different superscript are different (p<0.05)

The results indicate that the oocytes completed meiosis earlier if matured in the presence of luteinizing hormone (NIH-oLH) (66.0% at 16 hours) than in the presence of follicular stimulating hormone (FSHp) (26.5% at 16 hours).

### Claims

1. A process of maturing denuded bovine oocytes, comprising:
   a. isolating bovine cumulus oocyte complexes;
   b. placing the complexes in a maintenance medium;
   c. removing the cumulus cells from the oocytes to form denuded bovine oocytes; and
   d. maturing the denuded bovine oocytes in a conditioned medium containing a monolayer of cumulus cells.

2. The process according to claim 1 wherein the oocytes are matured in the conditioned medium for approximately 10-24 hours.

3. The process according to claim 1 wherein the oocytes are matured in the conditioned medium for approximately 16-20 hours.

4. The process according to claim 1 wherein the maintenance medium is selected from the group consisting of Ham's F-10 + 10% fetal calf serum (FCS), Tissue Culture Medium - 199 (TCM-199) + 10% FCS, Tyrodes-Albumin -Lactate-Pyruvate (TALP), Dulbecco's Phosphate Buffered Saline, Eagle's and Whitten's media, and CR1aa.

5. The process according to claim 4 wherein the maintenance medium includes serum supplement, pyruvate and gentamicin.

6. The process according to claim 4 wherein the maintenance medium includes fetal calf serum.

7. The process according to claim 1 comprising maturing the denuded bovine oocytes in the conditioned medium at a temperature of approximately 39°C, wherein the conditioned medium is a maintenance medium containing a monolayer of cumulus cells.

8. A process for selecting bovine oocytes for genetic manipulation processes, comprising:
   a. isolating bovine cumulus oocyte complexes;
   b. placing the complexes in a maintenance medium;
   c. removing the cumulus cells from the oocytes to form denuded bovine oocytes;
   d. maturing the denuded bovine oocytes in a conditioned medium containing a monolayer of cumulus cells;
   e. observing the presence of a first polar body on the denuded bovine oocyte; and
   f. selecting bovine oocytes based upon the observations of step e.

9. The process according to claim 8 wherein the oocytes are matured in the conditioned medium for approximately 10-24 hours.

10. The process according to claim 8 wherein the oocytes are matured in the conditioned medium for approximately 16-20 hours.

11. The process according to claim 8 wherein the complexes are maintained in the maintenance medium for approximately .5 hours before the cumulus cells are removed therefrom.

12. The process according to claim 8 wherein the genetic manipulation processes include nuclear transfer, gene transfer, embryo cloning, and genetic alteration by gene transfer.

13. A process for selecting bovine oocytes for genetic manipulation purposes, comprising:
   a. isolating bovine cumulus enclosed oocytes;
   b. maturing the cumulus enclosed oocytes in a maintenance medium including luteinizing hormone for approximately 10-24 hours;
   c. removing the cumulus cells from the oocytes to form denuded bovine oocytes;
   d. observing the presence of a first polar body on the denuded bovine oocytes; and
   e. selecting bovine oocytes based upon the observations of step d.

14. The process according to claim 13 wherein the oocytes are matured in the medium for approximately 16-20 hours.

15. The process of claim 13 wherein the luteinizing hormone is present in an amount ranging from about 2 $\mu$g/ml to 10 $\mu$g/ml.

16. The process of claim 13 wherein approximately 5 $\mu$g/ml luteinizing hormone is present.

17. A conditioned medium for maturing denuded bovine oocytes comprising a maintenance medium having a monolayer of cumulus cells.

18. The conditioned medium of claim 17 wherein the maintenance medium is selected from the group consisting of Ham's F-10 + 10% fetal calf serum (FCS), Tissue Culture Medium - 199 (TC-199) + 10% FCS, Tyrodes-Albumin -Lactate-Pyruvate (TALP), Dulbecco's Phosphate Buffered Saline, Eagle's and Whitten's media, and CR1aa.

19. A process for forming a conditioned medium for _in vitro_ maturation of denuded bovine oocytes comprising:
   a. washing cumulus cells; and
   b. suspending the cumulus cells in a maintenance medium for a time sufficient to form a cumulus cell monolayer in the maintenance medium.

20. The process according to claim 19 wherein the cumulus cells are suspended in the maintenance medium for a period of at least approximately two days.

21. The process according to claim 19 wherein the cumulus cells are suspended in the maintenance medium for a period of approximately 2 - 4 days.

22. A process for selecting bovine oocytes of higher quality for genetic manipulation processes, comprising:
   a. isolating bovine cumulus oocyte complexes;
   b. placing the complexes in a maintenance medium;
   c. removing the cumulus cells from the oocytes to form denuded bovine oocytes;
   d. maturing the denuded bovine oocytes in a conditioned medium containing a monolayer of cumulus cells;
   e. observing the presence of a first polar body on the denuded bovine oocyte; and
   f. selecting bovine oocytes based upon the observations of step e.

23. The process according to claim 22 wherein the complexes are maintained in the maintenance medium for approximately .5 hours before the cumulus cells are removed therefrom.

24. The process according to claim 22 wherein the oocytes are matured in the conditioned medium for approximately 16-21 hours.

25. The process according to claim 22 wherein the genetic manipulation processes include nuclear transfer, gene transfer, embryo cloning, and genetic alteration by gene transfer.

FIG. 1

FIG. 2

FIG. 3

FIG. 4

**European Patent Office**

# EUROPEAN SEARCH REPORT

Application Number

EP 92 30 5952

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | THERIOGENOLOGY vol. 37, no. 1, 1992, page 203 DOMINKO, T. & FIRST, N.L. 'Kinetics of bovine oocyte maturation allows selection for developmental competence and is affected by gonadotropins' * the whole document * | 13-16 | C12N5/06 A01K61/02 C12N5/00 |
| D,A | DEVELOPMENTAL BIOLOGY vol. 102, no. 2, February 1984, pages 493 - 497 SCHROEDER, A.C. & EPPIG, J.J. 'The developmental capacity of mouse oocytes that matured spontaneously in vitro is normal' * the whole document * | 1 | |
| X | EP-A-0 340 934 (UNIVERSITY COLLEGE OF DUBLIN) * page 3, column 4, line 27 - page 4, column 6, line 55 * | 1,2,17, 19 | |
| Y | * page 6, column 10, line 16 - page 7, column 11, line 51; claims 1,10,11 * | 1-3, 7-10, 12-14, 22,24,25 | TECHNICAL FIELDS SEARCHED (Int. Cl.5) C12N A01K |
| Y,D | WO-A-9 013 627 (GRANADA BIOSCIENCES) * the whole document * | 1-3, 7-10, 12-14, 22,24,25 | |

The present search report has been drawn up for all claims

| Place of search | Date of completion of the search | Examiner |
|---|---|---|
| THE HAGUE | 20 OCTOBER 1992 | CHAMBONNET F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)